# EUROPEAN PATENT APPLICATION

(11) **EP 1 767 208 A1**
(43) Date of publication of application: **28.03.2007**
(21) Application number: 05765484.0
(22) Date of filing: 05.07.2005
(51) Int. Cl.: A61K 31/455, A61P 13/10, A61P 13/00, A61P 43/00, A61P 9/08, C07D 213/82

(54) **REMEDY FOR HYPERACTIVE BLADDER**

(30) Priority: 05.07.2004 JP 2004226177
(71) Applicant: CHUGAI SEIYAKU KABUSHIKI KAISHA, Tokyo, 115-8543 (JP)
(72) Inventor: HORIE, Shigeo, 1650026 (JP); SAITO, Keiji, c/o Chugai Seiyaku Kabushiki Kaisha, Gotenba-shi, Shizuoka 4128513 (JP); ISHIZUKA, Nobuhiko, c/o Chugai Seiyaku K. K., Gotenba-shi, Shizuoka 4128513 (JP)
(74) Representative: Berryman, Natalia Grace
(86) International application number: PCT/JP2005/012402
(87) International publication number: WO 2006/004115

(57) **Abstract**

The present invention provides a therapeutic agent for overactive bladder, which has both a K_{ATP} channel opening action and a nitrate-like action and is further expected to produce an ameliorating effect for ensuring a reflexive and suitable cooperation between contraction-relaxation of abdominal/bladder wall muscle and relaxation-contraction movement of urethral sphincter during urination and urine storage, so that the therapeutic agent does not induce oliguria or urinary retention and has no serious side effect.

Nicorandil or a pharmaceutically acceptable salt thereof is used as an active ingredient.

## Description

### TECHNICAL FIELD

The present invention relates to a therapeutic agent for overactive bladder which comprises nicorandil as an active ingredient. The present invention relates to a therapeutic agent for overactive bladder, particularly to medication for frequent urination and urinary incontinence associated with urinary urgency.

### BACKGROUND ART

In the field of urology, a medical condition called overactive bladder has appeared to have little relation to vital prognosis and has sometimes tended to be neglected. However, this condition is known to cause a significant loss of the patient's social activity and/or QOL because it leads to serious symptoms such as a sense of urinary urgency, frequent urination and urinary incontinence. Patients who complain of such frequent urination symptoms number between 7,000,000 and 8,000,000 in Japan, including predominantly the elderly. As Japan has been graying now, the number of patients increase more and more. In these days when Japan has entered an aging society, not only the contribution to life support, but also the contribution to improved quality of life becomes considered to be an important mission in medical science and medical care.

Pathogenesis of frequent urination and urinary incontinence (urge type), results from bladder Overactivity which is considered as a pathological condition in which the urinary bladder spontaneously contracts during urine storage, i.e., involuntary contraction of bladder smooth muscle(Non-patent Document 1). Possible mechanisms for this pathogenesis include: (1) brain disorders superior to the micturition center (cerebrovascular disease type) and spinal cord disorders superior to the sacral spinal cord (e.g., spinal cord injury, multiple sclerosis); (2) patients with lower urinary tract obstruction (benign prostatic hyperplasia); (3) elderly people; (4) bladder hypersensitivity (e.g., chronic cystitis, interstitial cystitis); and (5) essential overactive bladder. Among cases of overactive bladder, the largest number of patients are idiopathic type (or mixed type) of unknown etiology. Also, there is a report of neurotransmitter release from postganglionic neurons, which shows that ATP release increases with aging, whereas acetylcholine (ACh) release decreases with aging. These findings suggest that an anticholinergic drug alone is not sufficient to ameliorate involuntary contraction of the urinary bladder of the eldery patient, and hence it is necessary to use an additional agent having a relaxing effect on smooth muscle. Also, there has been a problem of poor medication compliance arising from serious side effects (e.g., oliguria, urinary retention) caused by administration of an anticholinergic drug, as well as dry mouth, constipation, blurred vision, etc.

Previous studies have reported pharmacological effects against frequent urination using openers of ATP-sensitive potassium channels (hereinafter abbreviated as "K_{ATP} channel"). Such effects are considered to have the clinical purpose of relaxing detrusor muscle to increase intravesical urine storage (i.e., the volume of urine stored in the urinary bladder), thereby reducing the frequency of urination.

In recent years, K_{ATP} channel openers have been reported to have a relaxing effect on urinary bladder smooth muscle (see, e.g., Patent Document 1) and efforts have been made to develop them further. K_{ATP} channel openers developed previously have been found to have a useful therapeutic effect on overactive bladder due to relaxation of urinary bladder smooth muscle (Non-patent Documents 2 and 3).

On the other hand, co-relaxation of bladder neck sphincter and urethral sphincter is observed at the initiation of urination. This reaction is non-adrenergic and non-cholinergic (NANC). NANC nerves are considered to play a major role in urethra relaxation during urination. NANC nerves that play a modulatory role in smooth muscle tone in peripheral tissues including the cardiovascular system are typified by nitrenergic (NO) nerves that cause a relaxation response. In the lower urinary tract, nitric oxide synthase (NOS) activity is reported to be higher in the prostate and bladder neck than in the detrusor muscle (Non-patent Document 4). In the urinary bladder, NO is considered to have no direct relaxing effect on the bladder smooth muscle, but to indirectly relate to relaxation of the urinary bladder and inhibition of its activity during urine storage by acting on cholinergic nerves to reduce the amount of ACh release. Moreover, a reduction in NO release is observed in patients with lower urinary tract symptoms resulting from aging or benign prostatic hyperplasia, and it is deduced that this constitutes a possible cause of dynamic obstruction in the prostatic urethra (Non-patent Document 5). It is also suggested that NO released from the urothelium may be a possible mediator of detrusor muscle relaxation during urine storage (Non-patent Document 6).

Thus, some effect on overactive bladder medication can be expected even for pharmaceutical agents which show either a K_{ATP} channel opening action or a nitrate-like action alone. However, in view of the fact that contraction-relaxation of urinary bladder wall is reflexively cooperated with relaxation-contraction movement of urethral sphincter, these actions may be expected to produce a further synergistic effect when used in combination. Attention was therefore directed to substances having both a K_{ATP} channel opening action and a nitrate-like action.

Examples known as substances having both a K_{ATP} channel opening action and a nitrate-like action include nicorandil and KRN2391 (Non-patent Document 7). Since they have both two actions, they can be expected to show a higher effect that cannot be achieved by a K_{ATP} channel opening action alone. Namely, these substances were expected not only to cause urinary bladder relaxation and detrusor muscle relaxation due to their direct relaxing effect (mainly K_{ATP} channel opening action), but also to produce an effect on the bladder neck sphincter or prostate due to their ameliorating effect on neurotransmission disorders (mainly nitrate-like action). In fact, it has already been reported that KRN2391 has an ameliorating effect on frequent urination (Non-patent Document 8).

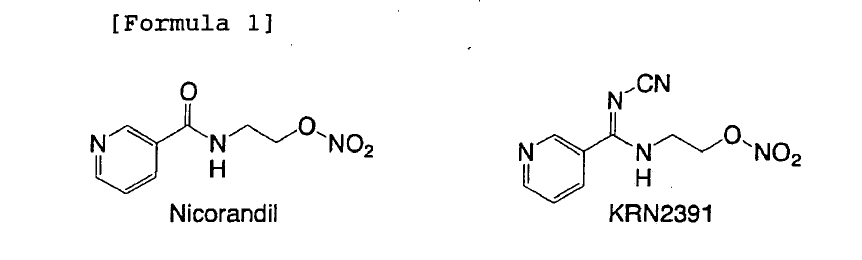

However, as shown in the data described later, it has actually become apparent that KRN2391 has an unwanted effect at some unforeseen point.

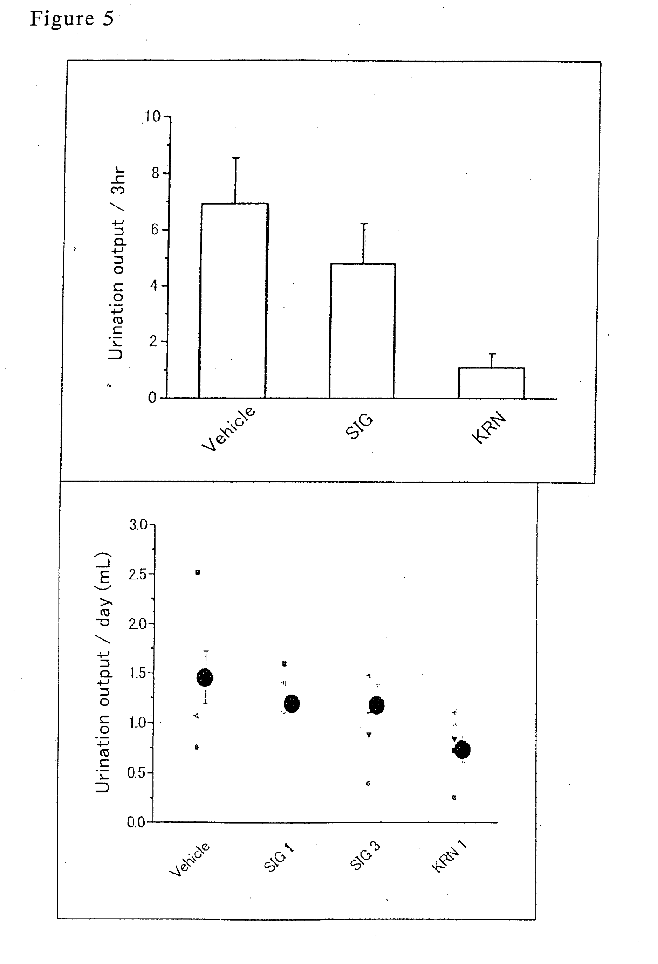

Namely, KRN2391 was found to have a tendency to cause not only excessive control of urination volume after water loading, but also more control of daily urination volume than required (Figure 5). It is therefore considered to have a possibility of developing oliguria and urinary retention, or to provide a sense of being oppressed, an excessive relaxing effect, incomplete urination, a sense of residual urine and the like. It is also considered to require much time to restore the urinary bladder to its normal level. Thus, KRN2391 may cause a loss of the patient's social activity and/or QOL.

KRN2391 further has a strong hypotensive effect and hence was expected to cause serious side effects on the cardiovascular system.

On the other hand, N-(2-hydroxyethyl)nicotinamide nitrate (common name: nicorandil) is a known compound that has been commercially available as an antianginal drug for a long time, and can be found in, e.g., Patent Document 2. Nicorandil is known to have some other effects in addition to an antianginal effect. For example, its effect as a therapeutic agent for cardiovascular diseases is disclosed in Patent Document 3, and its bronchodilating effect in Patent Document 4. In addition, Patent Document 5 discloses that nicorandil is useful as a therapeutic agent for diseases associated with cerebral ischemic lesions, while Patent Document 6 shows that nicorandil is useful as a hydroxyl radical scavenger. Recently, its effect as a therapeutic agent for anxiety neurosis has been disclosed in Patent Document 7. This document also shows that frequent urination is among the symptoms of anxiety neurosis. However, it is not known that nicorandil has an ameliorating effect particularly on overactive bladder-induced frequent urination.
[Non-patent Document 1] Osamu Yamaguchi, Folia Pharmacol. Jpn, 2003, 121:331-8
[Patent Document 1] JP 2003-506355 A
[Non-patent Document 2] Andersson, Urology, 1997, 50 (Suppl 6A), 74-84
[Non-patent Document 3] Lawson, Pharmacol Ther, 1996, 70, 39-63
[Non-patent Document 4] Ehren Urology, 1994, 44:683
[Non-patent Document 5] Masaki Yoshida, Folia Pharmacol. Jpn, 2003, 121:307-16
[Non-patent Document 6] Theobald RJ, Neurourol Urodyn, 2003, 22(1):62-9
[Non-patent Document 7] Muraki K. Brit, J Pharmac, 2001, 132, 1154-1160
[Non-patent Document 8] Kotani H, Jpn J Pharmacol, 1996, 71 (suppl 1), Abst p.221
[Patent Document 2] JP 52-122373 A
[Patent Document 3] JP 53-9323 A
[Patent Document 4] JP 58-85819 A
[Patent Document 5] JP 63-152317 A
[Patent Document 6] JP 3-101621 A
[Patent Document 7] JP 9-110695 A

### DISCLOSURE OF THE INVENTION

### PROBLEMS TO BE SOLVED BY THE INVENTION

Since the above prior art suffers from the problems mentioned above, it has been suggested that KRN2391 cannot actually be used as a drug in clinical cases. At present, there is no pharmaceutical agent having both a K_{ATP} channel opening action and a nitrate-like action, which has actually been marketed as a therapeutic agent for overactive bladder.

Under these circumstances, the object of the present invention is to provide a therapeutic agent for overactive bladder, which has both a K_{ATP} channel opening action and a nitrate-like action and is further expected to produce an ameliorating effect for ensuring a reflexive and suitable cooperation between contraction-relaxation of abdominal/bladder wall muscle and relaxation-contraction movement of urethral sphincter during urination and urine storage, so that the therapeutic agent does not induce oliguria or urinary retention and has no serious side effect.

As a result of extensive and intensive efforts made to achieve the above object, the inventors of the present invention have now found that nicorandil has a desired purpose, i.e., an ameliorating effect on urination disorders, which causes no excessive control of urination frequency and urination volume, and which does not induce oliguria or urinary retention. This finding led to the completion of the present invention.

Namely, the inventors of the present invention have found that nicorandil not only has an ameliorating effect (particularly a frequent urination-ameliorating effect) on overactive bladder-induced urination disorders, but also causes no excessive control of urination frequency and urination volume, thus producing an ameliorating effect for ensuring a cooperative movement between urination and urine storage. Moreover, its control effect on urination volume was not remarkably changed even at increased dosages. Thus, nicorandil has a very low possibility of inducing oliguria and urinary retention. Furthermore, its assuumed side effects on circulatory organs are very weak. Based on these results, the inventors have found that nicorandil is very effective as a therapeutic agent for urination disorders also in terms of the patient's social activity and QOL. In view of this finding, the inventors have found that the above object is achieved, and have arrived at the present invention.

### MEANS FOR SOLVING THE PROBLEMS

Namely, the present invention provides a therapeutic agent for overactive bladder which comprises nicorandil or a pharmaceutically acceptable salt thereof as an active ingredient.

The term "overactive bladder" is intended to mean, for example, overactive bladder induced by brain disorders, spinal cord disorders of the supranuclear type, benign prostatic hyperplasia, bladder hypersensitivity or aging, or alternatively, essential overactive bladder.

Likewise, overactive bladder-induced urination disorders are intended to mean, for example, a sense of urinary urgency, urinary incontinence, pollakiuria, detrusor instability, nocturnal enuresis, detrusor hyperreflexia and enuresis. In a case where overactive bladder-induced urination disorders are associated with a sense of urinary urgency, their symptoms include, for example, pollakiuria, nocturnal enuresis or urinary incontinence.

It should be noted that the above effect of nicorandil as a therapeutic agent for anxiety neurosis shown in Patent Document 7 is based on its pharmacological effects on the central nervous system. In contrast, the effect of nicorandil as a therapeutic agent for overactive bladder in the present invention is based on its K_{ATP} channel opening action and nitrate-like action in the urinary bladder, but not on its pharmacological effects on the central nervous system, and is further based on its action of restoring overactive bladder to an appropriate normal level. Thus, the present invention is completely different as a use invention from the invention shown in Patent Document 7.

The present invention provides a method for ameliorating or treating frequent urination, which uses a pharmaceutical composition comprising nicorandil or a pharmaceutically acceptable salt thereof as an active ingredient and which does not have, as a side effect, any hypotensive effect caused by its K_{ATP} channel opening action.

As defined herein, the term "nicorandil" refers to the common name of N-(2-hydroxyethyl)nicotinamide nitrate, which is represented by the following chemical formula (I).

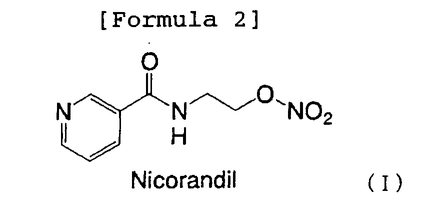

Nicorandil, a therapeutic ingredient in the present invention, is a compound commercially available as a therapeutic agent for angina pectoris. For use in the present invention, commercially available nicorandil tablets or injectable formulations may be used directly, or alternatively, nicorandil may also be prepared as described in JP 52-122373 A, etc.

Compound (I) may be either in free form or as a pharmaceutically acceptable salt, both of which fall within the scope of the present invention. Such a "salt" is not limited in any way as long as it can form a salt with Compound (I) of the present invention and is pharmaceutically acceptable. Examples include acid salts formed by reaction between Compound (I) of the present invention and an acid, as well as base salts formed by reaction between Compound (I) and a base.

Nicorandil, a therapeutic ingredient used in the present invention, can form an acid addition salt with a pharmaceutically acceptable organic or inorganic acid. Such a salt may also be used in the present invention. Examples of such an acid addition salt include a hydrochloride salt, a hydrogen bromide salt, a phosphate salt, a sulfate salt, a nitrate salt, an oxalate salt, a lactate salt, a tartrate salt, an acetate salt, a salicylate salt, a benzoate salt, a formate salt, a propionate salt, a pivalate salt, a diethylacetate salt, a malonate salt, a succinate salt, a pimelate salt, a fumarate salt, a maleate salt, a malate salt, a sulfamate salt, a phenylpropionate salt, a gluconate salt, an ascorbate salt, an isonicotinate salt, a methanesulfonate salt, a p-toluolsulfonate salt, a citrate salt, an adipate salt or a naphthalenedisulfonate salt.

In addition, when allowed to stand in ambient air, Compound (I) of the present invention may absorb water to have absorbed water molecules or to convert into a hydrate form. These cases also fall within the scope of the present invention.

Likewise, Compound (I) of the present invention may absorb a certain other solvent to convert into a solvate form, and such a salt also falls within the scope of the present invention. The term "solvation" as used herein is intended to mean a phenomenon in which solute molecules or ions in a solution strongly attract their adjacent solvent molecules to form a single molecular population. For example, if a solvent is water, this phenomenon is called hydration.

Compound (I) or a pharmaceutically acceptable salt thereof according to the present invention is useful as a therapeutic agent for overactive bladder, which has an excellent ameliorating effect on frequent urination and further has no remarkable hypotensive effect as a side effect. Compound (I) or a pharmaceutically acceptable salt thereof according to the present invention can also be expected to produce a therapeutic effect on other overactive bladder diseases, i.e., urinary incontinence, urinary urgency, detrusor instability, nocturia, enuresis and detrusor hyperreflexia, because it is predicted to rely on relaxation of urinary bladder smooth muscle and amelioration of NANC neurotransmission disorders.

### ADVANTAGES OF THE INVENTION

Nicorandil not only has an ameliorating effect (particularly a frequent urination-ameliorating effect) on overactive bladder-induced urination disorders, but also causes no excessive control of urination frequency and urination volume, thus producing an ameliorating effect for ensuring a cooperative movement between urination and urine storage. Moreover, since nicorandil causes no serious side effect when used as a therapeutic agent for overactive bladder, it is clinically very useful as a therapeutic agent for overactive bladder also in terms of the patient's social life and QOL.

### BRIEF DESCRIPTION OF DRAWINGS

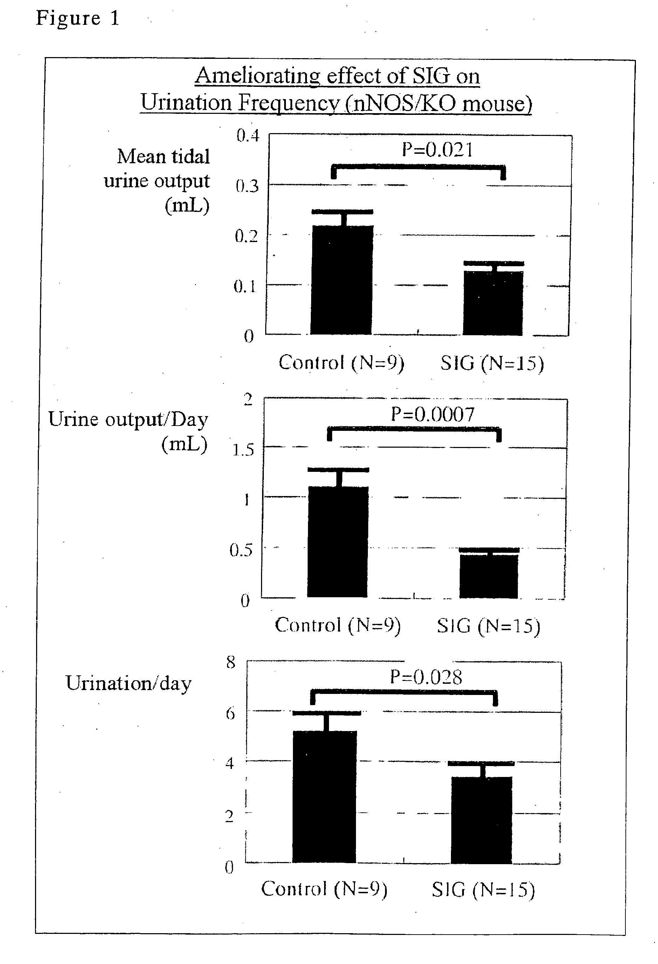

Figure 1 shows the effect of nicorandil administration on urination frequency in nNOS-Knockout mice. The mean ± standard error of each animal was plotted for the control group and the nicorandil group.

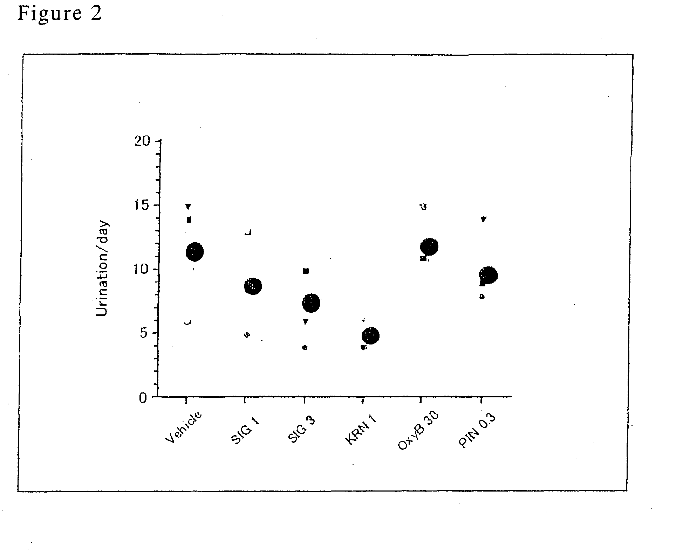

Figure 2 shows a comparison of nicorandil (SIG), KRN2391 (KRN), oxybutynin (OxyB) and pinacidil (PIN) for their ameliorating effect on frequent urination in nNOS-Knockout mice. The urination frequency of each animal was plotted for the control group (n = 8), the nicorandil groups (1 mg/kg (n = 8) and 3 mg/kg (n = 8)), the KRN2391 group (1 mg/kg, n = 6), the oxybutynin group (30 mg/kg, n = 6) and the pinacidil group (0.3 mg/kg, n = 5). The mean ± standard error of each group was indicated at the right side of each column.

It should be noted that each small symbol represents the individual result of each nNOS-Knockout mouse, while each large solid circle represents the mean value of each group.

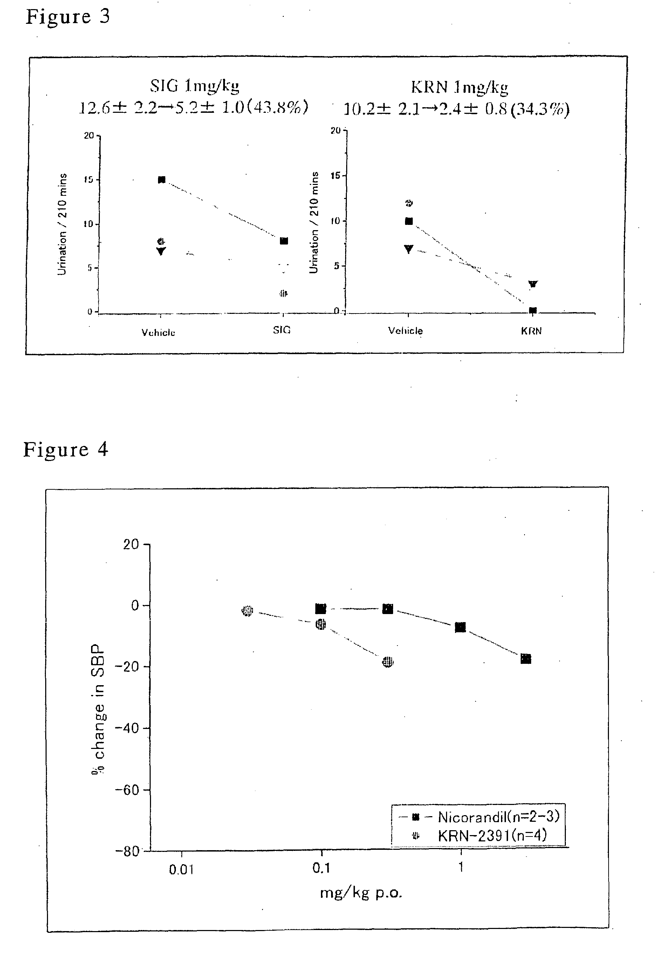

Figure 3 shows the inhibitory effect of nicorandil on frequent urination in a rat urethral obstruction model. At 45 days after urethral obstruction, rats with frequent urination (>6 times/210 minutes) were subjected to water loading (30 ml/kg, po) and measured for their urination frequency over 210 minutes after water loading. The results were compared among the vehicle group, the nicorandil group and the KRN2391 group (n = 5). The urination frequency of each animal was plotted for the control group (vehicle group), the nicorandil group and the KRN2391 group.

It should be noted that each symbol represents the individual result of each nNOS-Knockout mouse.
Figure 4 is a graph showing a comparison of nicorandil and KRN2391 for their hypotensive effect (systolic blood pressure (SBP)) in non-anesthetized conscious rats. The mean ± standard error of each animal was plotted for the nicorandil group and the KRN2391 group.
The upper panel of Figure 5 is a graph showing a comparison of nicorandil and KRN2391 for their control effect on urination volume after water loading in urethral obstruction rats. The lower panel of Figure 5 is a graph showing a comparison of nicorandil and KRN2391 for their control effect on daily urination volume.

It should be noted that each symbol represents the individual result of each nNOS-Knockout mouse.

### BEST MODE FOR CARRYING OUT THE INVENTION

Urination disorders in the context of the present invention are based on urinary bladder overactivity. Overactive bladder is induced by brain disorders, spinal cord disorders of the supranuclear type, benign prostatic hyperplasia, bladder hypersensitivity or aging, or alternatively, is essential overactive bladder. Symptoms observed in the urination disorders intended herein include a sense of urinary urgency, urinary incontinence, pollakiuria, detrusor instability, nocturnal enuresis, detrusor hyperreflexia and enuresis, as well as night urination. The pharmaceutical agent of the present invention which comprises, as an active ingredient, nicorandil having both a nitrate-like action and a K_{ATP} channel opening action is useful as a therapeutic and/or prophylactic agent for urination disorders, particularly frequent urination and night urination.

These medications involve the step of administering a pharmaceutically effective amount of a pharmaceutical composition comprising the disclosed Compound (I) or a pharmaceutically acceptable salt thereof to a patient who is in need of such medication or who suffers from such a disease or condition.

In a case where the pharmaceutical composition of the present invention is used as a therapeutic agent for overactive bladder, the mode of its administration includes oral, rectal, parenteral (intravenous, intramuscular, subcutaneous), intracisternal, intravaginal, intraperitoneal, intravesical or topical (in the form of drops, powders, ointments, gels or creams) administration, as well as inhalation (in the form of oral or nasal sprays).
In the present invention, when used as a therapeutic agent for urination disorders, particularly for amelioration of frequent urination, nicorandil or a pharmaceutically acceptable salt thereof is preferably formulated into an appropriate dosage form and, for example, can be used in the form of a formulation such as a tablet, a powder, a granule, a fine granule, a capsule, an injection, an emulsion, a suspension, a suppository or a percutaneous absorption preparation.

These formulations may be prepared as described in, e.g., JP 57-145659 A, JP 58-39618 A, JP 61-143316 A, JP 62-149630 A, JP 62-161727 A, JP 62-252722 A, JP 62-252723 A, JP 63-270624 A, etc. In more detail, for use as a tablet, nicorandil or a salt thereof may be incorporated with an organic acid (e.g., fumaric acid, oxalic acid, salicylic acid, tartaric acid, glutaric acid) and a mixture of one or more members selected from saturated higher fatty acids that are solid at an atmospheric temperature (e.g., stearic acid, palmitic acid) or saturated higher alcohols (e.g., cetyl alcohol, stearyl alcohol), or alternatively, may be blended with fumaric acid and/or DL-tryptophan, by way of example. As an example of how to prepare nicorandil tablets, the tablets can be prepared as described in JP 9-110695 A, etc.

Likewise, for use as an injection, nicorandil or a salt thereof may be formulated into a non-solution type injection by incorporation with an alkali metal salt of an organic acid (e.g., citric acid, fumaric acid, oxalic acid, malonic acid, maleic acid, tartaric acid). In the case of preparing these formulations, it is preferable to further incorporate a commonly used pharmaceutically acceptable carrier(s) such as an excipient, a disintegrating agent, a lubricant, a binder, a flavoring agent and/or a coloring agent. Examples of such a carrier include lactose, corn starch, mannitol, kaolin, crystalline cellulose, hydroxypropyl cellulose, hydroxypropylmethyl cellulose, carboxymethyl cellulose, carboxymethyl cellulose calcium, talc, croscarmellose sodium, anhydrous calcium hydrogen phosphate, calcium carbonate, calcium citrate, calcium stearate, and magnesium stearate.

In the present invention, the dosage of nicorandil or a salt thereof may be selected as appropriate for the condition, physique, diathesis, age and sex of a patient to be administered, the intended route of administration, the type of dosage form, etc. In general, for oral administration, the lower limit of dosage may be selected from the range of 1 to 15 mg/day, preferably 5 to 10 mg/day, and more preferably about 7.5 mg/day, while the upper limit of dosage may be selected from the range of 20 to 80 mg/day, preferably 25 to 60 mg/day, and more preferably about 30 mg/day, given in one to four divided doses by oral route. For parenteral administration, the lower limit of dosage may be selected as appropriate from the range of 0.1 to 12 mg/day, preferably 0.5 to 6 mg/day, and more preferably 1 to 2 mg/day, while the upper limit may be selected from the range of 10 to 50 mg/day, preferably 20 to 30 mg/day, and more preferably about 24 mg/day, given in one to four divided doses. Alternatively, it is also possible to administer 12 to 288 mg per day by sustained intravenous injection.

The nicorandil formulation of the present invention may further comprise one or more other therapeutic agents for urination disorders or other acceptable pharmaceutical agents, or alternatively, may be used in combination with these pharmaceutical agents. Appropriate ingredients desired for this purpose or other therapeutic agents include those commonly used for treating a condition of this nature. Above all, the nicorandil formulation of the present invention may be used in combination with a previously used pharmaceutical agent(s) that can be expected to produce an effect on diseases whose major symptom is frequent urination, as exemplified by a urinary bladder smooth muscle relaxant [flavoxate hydrochloride (trade name: Bladderon, Nippon Shinyaku Co., Ltd., Japan)], a β2 adrenaline stimulant [clenbuterol hydrochloride (trade name: Spiropent, Teijin Limited, Japan)], an α1 blocker [urapidil (trade name: Ebrantil, Kaken Pharmaceutical Co., Ltd., Japan), etc.], and an anticholinergic drug [oxybutynin hydrochloride (trade name: Pollakisu, Aventis Pharma), propiverine hydrochloride (trade name: BUP-4, Taiho Pharmaceutical Co. Ltd., Japan and UCB Japan, Co., Ltd., Japan), tolterodine tartrate (trade name: Detrol, Pfizer Inc.)]. The nicorandil formulation of the present invention may further be used in combination with gene therapy.

Therapeutic agents for urination disorders listed herein include therapeutic agents for excretory disorders (e.g., cholinergic drugs, cholinesterase inhibitors), drugs acting on urethra smooth muscle (e.g., α1 blockers), drugs acting on rhabdosphincter (e.g., central muscle relaxants), and pharmaceutical agents with other mechanisms of action (e.g., antiandrogen formulations). Likewise, therapeutic agents for urine storage disorders include drugs acting on urinary bladder detrusor muscle (e.g., therapeutic agents for frequent urination and urinary incontinence, antianxiety drugs, tricyclic antidepressants), drugs acting on urethra smooth muscle (e.g., α agonists, sympathetic activators), drugs acting on rhabdosphincter (e.g., β2 adrenaline stimulants), and other pharmaceutical agents (e.g., central muscle relaxants, afferent nerve blockers).

As defined herein, the term "therapeutic" or "medication" refers to a treatment intended to facilitate functional restoration to normal levels, which is achieved by direct or indirect action of the present invention on a mechanism responsible for a target disease.

As defined herein, the term "frequent urination" refers to a condition showing an abnormally increased urination frequency, as compared to normal subjects who have 6 to 8 urinations per day and usually have no urination while sleeping in the night.

As defined herein, the term "frequent urination-ameliorating effect" refers to a condition where urination functions approach normal levels, which is achieved by direct or indirect action of the present invention on a mechanism responsible for frequent urination.

As defined herein, the term "inhibitory effect on frequent urination" refers to a condition where frequent urination symptoms are controlled to restore a normal state, which is achieved by direct or indirect action of the present invention on a mechanism responsible for frequent urination in the Examples using an animal model of frequent urination, in order to prove the present invention.

As defined herein, the term "urethral obstruction" refers to a condition where the proximal urethra physically experiences insufficient dilation to limit urinary excretion during urination in overactive bladder showing symptoms of dysuria-induced frequent urination.

As defined herein, the term "overactive bladder" is a disease concept proposed in 2002 by the International Continence Society (ICS) and means the sense of urinary urgency associated with frequent urination and/or night urination, regardless of the presence or absence of urge urinary incontinence.

As defined herein, the term "bladder hypersensitivity" refers to a condition where the urinary bladder becomes overactive as a result of overactivity of afferent sensory nerve from the urinary bladder due to inflammatory changes (e.g., chronic cystitis, interstitial cystitis) in the urinary bladder.

As defined herein, the term "essential overactive bladder" refers to a condition of idiopathic overactive bladder of unidentified etiology, in which two mechanisms, i.e., myogenic and neurogenic ones are involved simultaneously.

As defined herein, the term "urinary retention" refers to a condition where urine is difficult to excrete even though it exists in the urinary bladder, unless otherwise specified herein.

As defined herein, the term "oliguria" refers to a condition where the daily urination volume is reduced to 400 mL/day or less in human bladders, unless otherwise specified herein.

### EXAMPLES

The present invention will now be further illustrated by way of the following examples, which are not intended to limit the scope of the invention.

### Example 1 (Disease model)

There has been established no animal models of disease reflecting frequent urination in elderly male subjects. In carefully searching various kinds of information, nNOS-Knockout mice were pointed out to have a possibility as a model of frequent urination (dilatation of urinary bladder, hyperplasia of smooth muscle, lack of relaxation responses, see Non-patent Document 9 for its creation) in May, 1997 (Non-patent Document 10), but have not been studied as a therapeutic model. nNOS-Knockout mice have now been investigated for their possibility as a model of frequent urination by using an instrument (an observation system for urination behavior, Muromachi Kikai Co., Ltd., Japan) to determine urination dynamics (urination volume and urination frequency) of the mice.

As a result, dilatation of urinary bladder and hyperplasia of detrusor smooth muscle were observed in nNOS-Knockout mice (8-13 months of age, 30 g body weight, n = 9). As compared to normal animals (n = 8), these nNOS-Knockout mice were confirmed to show no change in single urination volume and daily urination volume, but a significant increase in urination frequency (p<0.01), thus finding that a nNOS-Knockout mouse could be used as an animal model of frequent urination. When the nNOS-Knockout animals were further administered with testosterone (Enarmon-depot 3.6 mg/kg/day, sc, 7 days, n = 5), which was considered to have an ameliorating effect on frequent urination, the animals showed no change in daily urination volume, but a significant increase in single urination volume (p<0.05) and a significant decrease in urination frequency (p<0.05), as compared to the control group (n = 8). In this test, testosterone showed a clear ameliorating effect on abnormal urination, thus proving that the animals were established as an animal model of frequent urination in humans.

In more detail, testosterone administered herein was an Enarmon-depot (common name: testosterone enanthate, Takeda Pharmaceutical Co., Ltd., Japan), which was given at 3.6 mg/kg per day by subcutaneous injection for 7 consecutive days.
[Non-patent Document 9] Huang PL, Cell, 1993 75:1273-86.
[Non-patent Document 10] Burnett AL, Nat Med, 1997 3:571-4

### Example 2 (Pharmacological effects)

The causes believed to induce frequent urination are hyperplasia or intensive contraction of urinary bladder smooth muscle, as well as disturbance of neurotransmission to urinary bladder smooth muscle. Since nicorandil has both a K_{ATP} channel opening action and a nitrate-like action, as shown above, attention was focused on its direct relaxing effect on urinary bladder smooth muscle (mainly K_{ATP} channel opening action) and its ameliorating effect on neurotransmission disturbance (mainly nitrate-like action). In this example, an investigation was started with the assumption that nicorandil would have a higher ameliorating effect on frequent urination than commonly used K_{ATP} channel openers.

Nicorandil was given to nNOS-Knockout mice (8-13 months of age) by forced oral administration at 6 mg/kg/day for 7 days using a sound for oral administration. The results obtained are shown in Figure 1. The urination frequency was significantly reduced from 5.22 times/day in the non-administered group (n = 9) to 3.47 times/day in the nicorandil group (n = 15) (p<0.05).
As indicated by the above results, nicorandil was found to cause a distinct reduction of urination frequency in nNOS-Knockout mice as an animal model of frequent urination in humans, and thus confirmed to have an ameliorating effect on frequent urination.

### Example 3 (Comparison of pharmacological effects)

KRN2391 is a vasodilator that is developed from and structurally similar to nicorandil used as a lead compound (Non-patent Documents 7, 11, 12 and 13). On the other hand, the effect of KRN2391 on urinary organs has also been reported (Non-patent Documents 8, 14, 15 and 16). When KRN2391 was used as a control drug to compare the effect on daily urination frequency in nNOS-Knockout mice, nicorandil (3 mg/kg) and KRN2391 (1 mg/kg) were found to show a significant ameliorating effect on urination (p<0.05, Figure 2).

The daily urination frequency was measured with an observation system for urination behavior (Muromachi Kikai Co., Ltd., Japan).
Further, oxybutynin hydrochloride (SIGMA); which was a conventional anticholinergic drug, was also used for comparison of the urination frequency (Figure 2).

Furthermore, with the aim of clarifying the contribution of the nicorandil's nitrate-like action, pinacidil (SIGMA) known as a pure and potent K_{ATP} channel opener was also used for comparison of the urination frequency (Figure 2). When administered orally at a dose strongly affecting the mouse cardiovascular system (0.3 mg/kg), pinacidil showed no significant decrease in urination frequency.

As indicated by the above results, in nNOS-Knockout mice as an animal model of frequent urination pathologically similar to human condition, nicorandil was confirmed to cause a reduction of urination frequency at a one-third level as compared to KRN2391. Likewise, when compared to oxybutynin as an anticholinergic drug, nicorandil was also confirmed to cause a reduction of urination frequency at a 10-fold or more level.

Moreover, no significant ameliorating effect on urination was observed in the K_{ATP} channel opener pinacidil even at a dose affecting the cardiovascular system. However, nicorandil showed an ameliorating effect on urination. This confirmed that having both a K_{ATP} channel opening action and a nitrate-like action was important in medication for overactive bladder with nicorandil.
[Non-patent Document 11] Ishibashi T. et al., Archives Pharmac 1992, 346, 94-1-101
[Non-patent Document 12] Takaharu Ishibashi et al., Kekkan (Blood Vessels) 1993, 16, 61-71
[Non-patent Document 13] Ishibashi T. and Imai S. Caeduivascular Drug Ther 1994, 12, 136-151
[Non-patent Document 14] Nakamura Y. et al., Urol 2002, 168, 2275-2279
[Non-patent Document 15] Waldeck K. et al., Gen Pharmacol 1995, 26, 1559
[Non-patent Document 16] Kotani H. et al., Jpn J Pharmacol 1999, 80, 143-153

### Example 4 (Comparison of pharmacological effects)

As described above, there are many causes that induce frequent urination, and dysuria-induced frequent urination is also known. A rat animal model of urethral obstruction has been reported as a model for efficacy verification of many ameliorating agents for frequent urination (Non-patent Documents 17 and 18). In this example, a midline incision was made in the lower quadrant of each rat (9 weeks of age) under pentobarbital anesthesia, and the proximal urethra was ligatured together with a stainless rod (φ 1 mm) by using a 3-0 silk suture, followed by pulling out the rod to create a urethral obstruction model. At 45 days after urethral obstruction, the rats were subjected to water loading (30 ml/kg, po; "water loading" as used herein refers to forced oral administration of distilled water using a sound) and observed for their urination frequency over 210 minutes, such that animals having a urination frequency greater than that of normal animals (5 times) were selected as animals with frequent urination. These animals were administered orally with nicorandil (1 mg/kg) or KRN2391 (1 mg/kg) (dissolved in distilled water for water loading) and then observed for their urination frequency over 210 minutes (n = 5 for each group). As a result, the urination frequency was reduced from 12.6 ± 2.2 times/210 minutes to 5.2 ± 1.0 times/210 minutes for nicorandil administration (p<0.05, 43.8% reduction) and reduced from 10.2 ± 2.1 times/210 minutes to 2.4 ± 0.8 times/210 minutes for KRN2391 administration (p<0.05, 34.3% reduction) (Figure 3).

As indicated by the above results, in urethral obstruction rats as an animal model of frequent urination in humans, nicorandil was confirmed to cause a reduction of urination frequency at the same level as compared to KRN2391.
[Non-patent Document 17] Wojdan A et al, J Pharmac Exp Ther 1999 289:1410-18
[Non-patent Document 18] Pandita RK et al, J Urol 1999 162:943-8

### xExample 5 (Comparison of side effects)

There is a report showing that KRN2391 has a hypotensive effect due to its K_{ATP} channel opening action that is 25-fold stronger than that of nicorandil (Non-patent Document 7). In view of this fact, to study a hypotensive effect regarded as a side effect in the amelioration of frequent urination, both drugs were compared for their effect on blood pressure when orally administrated to non-anesthetized conscious rats ("non-anesthetized conscious rat" as used herein refers to an animal pre-treated such that a catheter for blood pressure measurement is inserted and placed in its femoral artery under anesthesia to ensure blood pressure monitoring, which animal is used on the following day after the anesthesia wears off). In this test, oral administration was accomplished by forced oral administration of a KRN2391 or nicorandil solution using a sound. The measurement of blood pressure was performed as follows: the catheter for blood pressure measurement was connected to a blood pressure transducer (Nihon Kohden Corporation, Japan) and input into a polygraph (Nihon Kohden Corporation, Japan). The results indicated that when compared at a dosage causing only a 20 mmHg reduction in the blood pressure, KRN2391 produced a hypotensive effect in a 10-fold smaller amount (Figure 4).

As indicated by the above results, nicorandil was confirmed to have a side effect dose that was one-tenth that of KRN2391 with respect to the hypotensive effect in rats which was a side effect in the amelioration of frequent urination.

### Example 6 (Comparison of side effects: urinary retention and oliguria)

"Urinary retention" as used herein refers to a state where the urination volume is 3 hr = 0.

In patients with frequent urination, urinary retention caused by administration of an anticholinergic drug is considered to be a serious side effect. In this example, an investigation was performed with the assumption that the action of inducing oliguria and urinary retention would be caused by long-term prevention of an increase in intravesical pressure due to administration of KRN2391 having a strong direct relaxing effect on urinary bladder smooth muscle (mainly a K_{ATP} channel opening action).

Rats at 45 days after urethral obstruction (φ 1 mm, urethra was obstructed in rats at 9 weeks of age) were administered orally with nicorandil (3 mg/kg) or KRN2391 (1 mg/kg), subjected to water loading (30 ml/kg, po) and then monitored for their urination volume over 180 minutes (n = 10 for each group). As a result, as shown in the upper panel of Figure 5, the urination volume (and the number of animals without urination) was 3.47 ± 0.65 mL (0 animals without urination) in the vehicle group and 1.86 ± 0.48 mL (0 animals without urination) in the nicorandil group, whereas the urination volume in the KRN2391 group was 0.50 ± 0.30 ml (5 animals without urination). In the KRN2391 group, no urination, i.e., a state of urinary retention was observed. Likewise, the KRN2391 group showed a smaller urination volume than the nicorandil group and hence was in a state of oliguria.

Moreover, as shown in the lower panel of Figure 5, when KRN2391 was used as a control drug to compare the effect on daily urination volume in nNOS-Knockout mice (12-16 weeks of age), nicorandil (3 mg/kg) had no significant influence, but KRN2391 (1 mg/kg) showed a significant inhibitory effect on daily urination volume (oliguria) (p<0.05). The figure shows the results obtained at 24 hours after administration of these drugs.

As indicated by the above results, nicorandil was confirmed to cause weaker side effects than KRN2391 with respect to the action of inducing oliguria and urinary retention in rats which were side effects in the amelioration of frequent urination.

### INDUSTRIAL APPLICABILITY

As shown in Examples 1 to 3, therapeutic agents for frequent urination comprising nicorandil of the present invention as a major ingredient can be expected to produce a therapeutic effect on frequent urination which is a symptom of overactive bladder. Nicorandil is advantageous as a therapeutic agent for overactive bladder in that its effect is substantially the same level as that of KRN2391 having the same structure, while it has very weak side effects including a hypotensive effect, urinary retention and oliguria, as shown in Examples 4 to 6.

Since the effect of nicorandil is predicted to be based on relaxation of urinary bladder smooth muscle and amelioration of NANC neurotransmission disturbance, nicorandil can also be expected to produce a therapeutic effect on other overactive bladder diseases, i.e., urinary incontinence, urinary urgency, detrusor instability, nocturia, enuresis and detrusor hyperreflexia. Medication based on the present invention is safe and effective as compared to medication with conventional anticholinergic drugs (oxybutynin, propiverine, tolterodine) or β stimulants (clenbuterol). Moreover, nicorandil not only has a smaller influence on the cardiovascular system than other K channel (i.e., K_{Ca} channel) openers known to have an effect on frequent urination, but also has an excellent effect which is free from tolerance to repeated use as compared to nitrate drugs.

## Claims

1. Use of nicorandil or a pharmaceutically acceptable salt thereof for the manufacture of a medicament for the treatment of overactive bladder.

2. The use according to claim 1, wherein the overactive bladder is induced by brain disorders, spinal cord disorders of the supranuclear type, benign prostatic hyperplasia, bladder hypersensitivity or aging, or alternatively, is essential overactive bladder.

3. The use according to claim 2, wherein a urination disorder induced by overactive bladder is a sense of urinary urgency, urinary incontinence, pollakiuria, detrusor instability, nocturnal enuresis, detrusor hyperreflexia or enuresis.

4. The use according to claim 3, wherein the urination disorder induced by overactive bladder is associated with a sense of urinary urgency and also associated with pollakiuria, nocturnal enuresis or urinary incontinence as a symptom.
